# EUROPÄISCHE PATENTSCHRIFT

(11) **EP 3 525 727 B1**
(45) Veröffentlichungstag und Bekanntmachung des Hinweises auf die Patenterteilung: **29.11.2023**
(21) Anmeldenummer: 18709020.4
(22) Anmeldetag: 09.03.2018
(51) Int. Cl.: A61F 2/42, A61B 17/80, A61F 2/30

(54) **ARTHRODESEPLATTE**
ARTHRODESIS PLATE
PLAQUE D'ARTHRODÈSE

(30) Priorität: 11.04.2017 EP 17165943
(43) Veröffentlichungstag der Anmeldung: 21.08.2019
(73) Patentinhaber: Waldemar Link GmbH & Co. KG, 22339 Hamburg (DE)
(72) Erfinder: LINK, Helmut D., 22397 Hamburg (DE); TEUBER, Jan, 22850 Norderstedt (DE)
(74) Vertreter: Alatis
(86) Internationale Anmeldenummer: PCT/EP2018/055899
(87) Internationale Veröffentlichungsnummer: WO 2018/188861

(56) Entgegenhaltungen:
- EP-A2- 2 158 864
- DE-U1-202009 008 872
- US-A1- 2007 156 241
- US-A1- 2010 125 301
- US-A1- 2013 066 435
- US-A1- 2016 367 300
- US-A1- 2017 007 416

## Beschreibung

Erfindungsgemäß ist eine Arthrodeseplatte, zur Gelenksversteifung insbesondere an Fingergelenken, mit einem proximalen und einem distalen Verankerungsbereich, die mechanisch starr miteinander verbunden sind.

In der orthopädischen und Unfallchirurgie finden Gelenksprothesen ebenso Anwendung wie Osteosyntheseplatten. Letztere werden in einer speziellen Anwendung auch zur geplanten Versteifung (Arthrodese) von beschädigten Gelenken verwendet. Die Anbringung solcher Implantate durch den Operateur sollte möglichst schnell, einfach und minimal invasiv sein, wobei auch Arthrodeseplatten so an den betroffenen Knochen zu verankern sein müssen, dass Kräfte zwischen Knochen und Implantat zuverlässig und dauerhaft übertragen werden.

Prothesen auch für Fingergelenke werden bekanntlich zum Beispiel im Markraum, ohne Schrauben, verankert. Implantate zur Arthrodese werden üblicherweise von außen an den Knochen geschraubt. Der für die genannten Verankerungsmethoden üblicherweise notwendige palmare oder dorsale Zugang birgt ferner die Gefahr, die Beuge- und/oder Strecksehnen zu verletzen.

Gleiches trifft auch für die Knochenfixations- bzw. Knochenfusionsvorrichtung der US 2016/0367300 A1 zu, welche die Grundlage für die zweiteilige Form bildet. Hier wird das Gelenk nicht seziert. Stattdessen umgreift die Vorrichtung dieser Offenbarungsschrift die durch das zu versteifende Gelenk getrennten Fingerknochen, um das Gelenk auf diese Weise zu versteifen. Die US 2010/0125301 A1 offenbart eine chirurgische Vorrichtung mit zwei Schenkeln und einer dazwischenliegenden Überbrückung, die ähnlich wie eine chirurgische Klammer wirkt.

Der vorliegenden Erfindung liegt die Aufgabe zu Grunde, eine Arthrodeseplatte insbesondere für Fingergelenke zu schaffen, die bezüglich ihrer Handhabung während der operativen Anbringung und bezüglich ihrer Verankerung verbessert ist.

Diese Aufgabe wird erfindungsgemäß von einer Arthrodeseplatte mit den Merkmalen des Anspruchs 1 gelöst. Vorteilhafte Ausgestaltungen sind in den Unteransprüchen angegeben.

Die erfindungsgemäße Arthrodeseplatte dient zur Gelenksversteifung insbesondere an Fingergelenken, aber auch an anderen Gelenken, die überwiegend Scharniergelenksfunktion haben. Sie weist einen proximalen und einen distalen Verankerungsbereich auf, die mechanisch starr miteinander verbunden sind. Erfindungsgemäß ist die Arthrodeseplatte dadurch gekennzeichnet, dass mindestens eine Außenkontur eines der beiden Verankerungsbereiche einen wellenförmigen Sagittal-Querschnitt hat (also einen wellenförmigen Querschnitt in einer Ebene, die parallel zur Sagittalebene liegt).

Im Sinne der vorliegenden Erfindung umfasst der Begriff "wellenförmig" neben stetigen (beispielsweise sinusförmigen) Wellenbergen und -tälern auch andere rastenförmige, zum Beispiel dachförmige oder sägezahnförmige Konturen. Das bedeutet, dass die Außenkontur des Verankerungsbereichs nicht unbedingt in runden, stetigen Wellenbergen und -tälern verlaufen muss. Stattdessen können die aufsteigenden und absteigenden Flanken der wellenförmigen Außenkontur auch geradlinig, bereichsweise geradlinig, selbst wellenförmig und/oder in beliebiger Weise unstetig ausgebildet sein. Anders ausgedrückt muss die Außenkontur des Verankerungsbereiches im Bereich seiner Hoch- und Tiefpunkte im geometrischen Sinne nicht stetig gekrümmt sein, sondern sie kann auch unstetig ausgebildet sein. Die Hoch- und Tiefpunkte der Außenkontur können also auch die Gestalt einer Kante haben, so dass der wellenförmige Querschnitt die Form einer Ecke oder Spitze einnimmt und eine punktförmige bzw. scharfkantige Richtungsänderung darstellt. So kann die erfindungsgemäß wellenförmige Außenkontur technisch zum Beispiel dadurch erzeugt werden, dass auf eine zunächst ebene Außenkontur eine Beschichtung aufgetragen wird, zum Beispiel mittels Plasma-Spray-Verfahren. Wird das Plasma-Spray-Verfahren so gesteuert, dass sich unterschiedliche Beschichtungsstärken an unterschiedlichen Stellen des Verankerungsbereiches einstellen, lässt sich eine wellenförmige Kontur erzeugen. Auch hier kann, abhängig von der Form der aufgetragenen Partikel, die Form eines Wellenbergs (also eines lokal höchsten Bereiches der Außenkontur) irgendwie gerundet, aber auch zum Beispiel eckig oder mit sonst wie unstetig gekrümmter Oberfläche ausgebildet sein. Entsprechendes gilt für die Wellentäler (also die lokal tiefsten Bereiche der Außenkontur).

Die erfindungsgemäße Arthrodeseplatte ist, in dieser Weise ausgestaltet, auf Folgendes eingerichtet und ermöglicht so die Verankerung eines erfindungsgemäßen Implantats (insbesondere auch für die Arthrodese eines distalen Interphalangealgelenkes oder eines proximalen Interphalangealgelenkes) in den mechanisch mittels der erfindungsgemäßen Arthrodeseplatte (zur Arthrodese starr) miteinander zu verbindenden Knochenenden auf folgende Weise: in das dem Gelenkdefekt zugewandte sowohl proximale als auch distale Knochenende (der durch das Implantat starr miteinander zu verbindenden Knochen) kann der Operateur (zum Beispiel mittels Fräse oder oszillierender Säge) einen (vorzugsweise in der Frontalebene des Fingerknochens liegenden und ungefähr durch dessen Mittenachse verlaufenden) ebenen Schlitz von medial oder lateral einbringen - der in die andere Richtung (lateral oder medial) besonders bevorzugt nicht ganz durch den Knochen hindurch geht. In diesen Schnitt kann nun der Operateur von derselben Seite (medial oder lateral) einen der beiden Verankerungsbereiche (mit vorzugsweise breiterem (in medio-lateraler Richtung) als hohem (in palmar-dorsaler Richtung), insbesondere flach rechteckigem Transversalquerschnitt)) einführen. Dabei hat er dort medial oder lateral am Finger nur weniger relevante Strukturen zu berücksichtigen. Er läuft also nicht Gefahr, wie bei herkömmlichen Implantaten die palmar oder dorsal liegenden Strukturen zu traumatisieren. Beim Einführen des Verankerungsbereichs von medial oder lateral in den ebenen Schlitz entsteht nun eine (insbesondere in der Sagittalebene) winkelstabile Verbindung zwischen dem Implantat und dem Knochenende - und überraschenderweise sogar auch eine gegen Auszugskräfte feste Verbindung - nämlich aufgrund von "Pressfit" (oder, in ingenieurstechnischen Begriffen, aufgrund von Presspassung): die Weite (Höhe) des ebenen Schlitzes ist dazu vom Operateur (geringfügig) kleiner auszubilden als die Höhe vorzugsweise mehrerer der (erfindungsgemäß transversal vorzugsweise weniger hoch als breit ausgebildeten) Querschnitte des Verankerungsbereichs. Diese soweit beschriebene Prozedur (Anbringen eines ebenen Schlitzes in das Knochenende; Einführen von medial oder lateral des Verankerungsbereichs in den ebenen Schlitz) wird am anderen Knochenende für den anderen Verankerungsbereich des Implantats - zum Anbringen des Implantats zur Arthrodese - wiederholt. Die auf diese Weise winkelstabil und auszugsfest mit den beiden Knochenenden verbundenen beiden Verankerungsbereiche sind erfindungsgemäß mechanisch starr miteinander verbunden, und zwar unter 180° (also in gerader Linie) zu einander oder alternativ in einem beliebigen Winkel (insbesondere 30°). In dieser Weise montiert, überbrückt das erfindungsgemäße Implantat die beiden Knochenenden starr zur Arthrodese.

So sind die beiden Verankerungsbereiche der erfindungsgemäßen Arthrodeseplatte insbesondere eingerichtet auf Verankerung in jeweils einem ebenen Schlitz (der von einem Operateur im zu behandelnden Knochen anzubringen ist), und zwar durch Pressfit (Presssitz oder Presspassung), indem nämlich (vorzugsweise mehrere) Querschnitte des jeweiligen Verankerungsbereichs eine größere Höhe aufweisen als die Höhe (Weite) des jeweiligen Schlitzes.

So ist die erfindungsgemäße Arthrodeseplatte also insbesondere eingerichtet für die Verankerung in je einem ebenen Schlitz in den beiden Knochenenden in der Frontalebene des Fingerknochens und ungefähr durch dessen Mittenachse verlaufend durch Pressfit. Diese Verankerung wird erfindungsgemäß weiter begünstigt dadurch, dass mindestens eine Außenkontur eines der beiden Verankerungsbereiche einen im Sinne der vorliegenden Erfindung wellenförmigen Sagittal-Querschnitt hat. Dieser kann zum Beispiel auch zickzackförmig sein mit geraden aufsteigenden Flanken und geraden absteigenden Flanken. Dieser wellenförmige Querschnitt ist insbesondere eingerichtet, mit seinen Wellenbergen in die Schnittflächen oder Grenzflächen des ebenen Schlitzes hineinzudrücken. Dies kann den Pressfit lokal, nämlich bei den Wellenbergen, verstärken - und zudem durch Hineindrücken der Wellenberge in die Schnittflächen auch für einen Formschluss sorgen.

Erfindungsgemäß bevorzugt ist zur Begünstigung der Pressfitverankerung eine Arthrodeseplatte, bei der sowohl die Oberseite als auch die Unterseite der Verankerungsbereiche insgesamt einen wellenförmigen Sagittal-Querschnitt haben - und sich so vollflächig (also vorzugsweise über die gesamte Länge in Längsrichtung des Implantats, nämlich üblicherweise in proximal-distaler Richtung und/oder über die gesamte Breite des Implantats, nämlich üblicherweise in lateraler Richtung) in die obere und untere Schnittfläche des ebenen Schlitzes eindrücken können.

Besonders bevorzugt ist es dann, dass der wellenförmige Sagittal-Querschnitt der Oberseite und der Unterseite mindestens abschnittsweise, vorzugsweise insgesamt, parallel zueinander verlaufen. So ergibt sich nicht nur ein wellenförmiger Sagittal-Querschnitt einer Verankerungsbereich-Außenkontur, sondern des Verankerungsbereichs insgesamt.

Diese und andere Merkmale der Erfindung werden im Folgenden mit Bezug auf die beigefügten Abbildungen von Ausführungsbeispielen der Erfindung beschrieben. Darin zeigen:
Figur 1a eine Darstellung einer erfindungsgemäßen Arthrodeseplatte in Seitenansicht von lateral auf eine Sagittalebene,
Figur 1b eine Darstellung der erfindungsgemäßen Arthrodeseplatte in Draufsicht von vorn auf eine Frontalebene,
Figur 1c eine geschnittene Darstellung der erfindungsgemäßen Arthrodeseplatte als Schnitt in einer Transversalebene in Ansicht von distal, und
Figur 2 eine räumliche Ansicht der erfindungsgemäßen Arthrodeseplatte nach Figur 1.

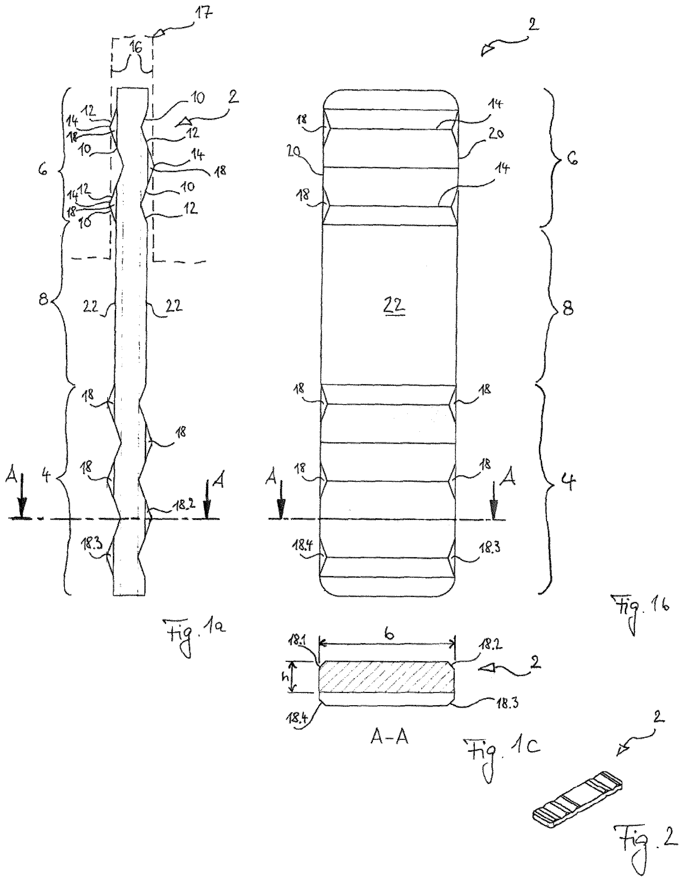

In den Figuren 1 und 2 ist eine Arthrodeseplatte 2 dargestellt. Die Arthrodeseplatte 2 dient zur Gelenksversteifung insbesondere an Fingergelenken (nicht dargestellt). Sie weist einen proximalen Verankerungsbereich 4 und einen distalen Verankerungsbereich 6 auf sowie einen Schaftbereich 8, der die beiden Verankerungsbereiche mechanisch starr miteinander verbindet. Die beiden Verankerungsbereiche 4, 6 haben transversale Querschnitte (einer ist in Figur 1c dargestellt), die breiter (b, in lateraler Richtung) sind als hoch (h, in palmar-dorsaler Richtung).

Die Außenkonturen 10, 12 der beiden Verankerungsbereiche 4, 6 haben insgesamt in Ebenen parallel zur Sagittalebene einen wellenförmigen Querschnitt (Figur 1a). Dieser ist zickzackförmig mit geraden aufsteigenden Flanken 10 und geraden absteigenden Flanken 12. Dieser wellenförmige Querschnitt ist insbesondere auch eingerichtet, mit seinen "Wellenbergen" 14 in die ebenen Schnittflächen 16 eines ebenen Schlitzes (17; dargestellt nur schematisch in gestrichelten Linien) hineinzudrücken. Dies kann dort lokal, nämlich bei den Wellenbergen 14, einen Pressfit begünstigen und zudem durch Hineindrücken der Wellenberge 14 in die Schnittflächen 16 auch für einen Formschluss mit dem Schlitz 17 sorgen. Sowohl die Oberseite als auch die Unterseite der Verankerungsbereiche 4, 6 haben insgesamt den wellenförmigen Sagittal-Querschnitt 10, 12: Oberseite und Unterseite verlaufen insgesamt fast vollständig parallel zueinander.

Jeder transversale Querschnitt (einer ist in Figur 1c dargestellt) der beiden Verankerungsbereiche 4, 6 ist rechteckig, wobei Ecken mancher der rechteckigen transversalen Querschnitte Fasen 18 (auch Radien (nicht dargestellt) sind erfindungsgemäße Alternativen) aufweisen, nämlich im Bereich sämtlicher Wellenberge 14.

Das Volumen der beiden Verankerungsbereiche 4, 6 ist in proximal-distaler Richtung geradlinig erstreckt mit geraden Seitenkanten 20 (Figur 1b). Auch das Volumen des Schaftbereichs erstreckt sich in proximal-distaler Richtung geradlinig mit geraden Seitenkanten 20 in einer Richtung und dabei fluchtend mit der Erstreckungsrichtung des Volumens der beiden Verankerungsbereiche, nämlich mit gemeinsamen geraden Seitenkanten 20. Alternativ (nicht dargestellt) kann der Schaftbereich in proximal-distaler Richtung einen Knick in seiner Erstreckung aufweisen, der die beiden Verankerungsbereiche in der Sagittalebene (Figur 1a) in einem Winkel zueinander orientiert (der vorzugsweise 1° bis 40° oder 5° bis 35° oder 30° sein kann insbesondere zur Arthrodese am proximalen Interphalangealgelenk - oder 1° bis 50° oder 5° bis 45° oder 30° zur Arthrodese am distalen Interphalangealgelenk). So ausgestaltet entsteht ein besonders zur Arthrodese der Interphalangealgelenke geeignetes Implantat, da in diesem Bereich Arthrodesen von Fingergelenken meist in so dimensionierter Beugestellung vorgenommen werden.

Das abgebildete Implantat 2 ist als Dauerimplantat vorgesehen und besteht zu diesem Zweck aus einer Titanlegierung. Die Oberflächen sind rau ausgebildet (Rₐ > 4), um Anlagerung und Anwachsen von Knochen zu begünstigen. Die Oberflächen sind mit einer wachstumsfördernden Beschichtung (nicht dargestellt; zum Beispiel Calciumsphosphat) beschichtet.

Das abgebildete Implantat 2 hat folgende Abmessungen, die sich für die Verwendung als Arthrodeseplatte am DIP-Gelenk bewährt haben. Die Stärke h des insgesamt flach rechteckigen Querschnitts (Figur 1c) des Implantats 2 beträgt 1,6 mm (vorzugsweise zwischen 1,4 und 1,8 mm), die Breite b beträgt 7 mm (vorzugsweise zwischen 6 und 8 mm). Die Gesamtlänge des Implantats 2 beträgt 26 mm (vorzugsweise zwischen 20 und 30 mm). Die Zähne oder Wellenberge 14 ragen über die Ebenen der Oberseiten 22 um 0,5 mm hinaus (vorzugsweise zwischen 0,2 und 1,0 mm), die Wellentäler sind 0,5 mm tief (vorzugsweise zwischen 0,1 und 1,0 mm). Die Flanken 10, 12 haben einen Neigungswinkel von 30° (vorzugsweise zwischen 20° und 40°) zu den Ebenen der Oberseiten 22 (Frontalebenen).

## Patentansprüche

1. Arthrodeseplatte (2) zur Gelenkversteifung, wobei die Arthrodeseplatte aufweist:
eine proximal-distale Richtung, eine Längsrichtung in der proximal-distalen Richtung und eine medio-laterale Richtung,
einen proximalen Verankerungsbereich (4) und einen distalen Verankerungsbereich (6), die mechanisch starr miteinander verbunden sind, wobei ein Transversalquerschnitt der Verankerungsbereiche zur Längsrichtung eine Höhe (h) und in der medio-lateralen Richtung eine Breite (b) aufweist, wobei die Breite größer ist als die Höhe,
**dadurch gekennzeichnet,**
**dass** der proximale Verankerungsbereich (4) und der distale Verankerungsbereich (6) in einer Ebene parallel zu der Sagittalebene und/oder in einer Transversalebene eine Außenkontur (10, 12) mit einem wellenförmigen Querschnitt aufweist, und
**dass** die Verankerungsbereiche (4, 6) in medio-lateraler Richtung in einen ebenen Schlitz (17) einführbar sind, wobei der wellenförmige Querschnitt eingerichtet ist, mit seinen Wellenbergen in die Grenzflächen des ebenen Schlitzes hineinzudrücken.

2. Arthrodeseplatte (2) nach Anspruch 1, **dadurch gekennzeichnet, dass** die Oberseite und die Unterseite der Verankerungsbereiche (4, 6) einen wellenförmigen Sagittal- und/oder Transversal-Querschnitt haben.

3. Arthrodeseplatte (2) nach Anspruch 2, **dadurch gekennzeichnet, dass** der wellenförmige Sagittal- und/oder Transversal-Querschnitt der Oberseite und der Unterseite mindestens abschnittsweise parallel zueinander verlaufen.

4. Arthrodeseplatte (2) nach einem der vorhergehenden Ansprüche, **dadurch gekennzeichnet, dass** der wellenförmige Sagittal- und/oder Transversal-Querschnitt zickzackförmig ist mit geraden aufsteigenden Flanken und geraden absteigenden Flanken.

5. Arthrodeseplatte (2) nach einem der vorhergehenden Ansprüche, **dadurch gekennzeichnet, dass** der transversale Querschnitt rechteckig ist.

6. Arthrodeseplatte (2) nach Anspruch 5, **dadurch gekennzeichnet, dass** Ecken des rechteckigen transversalen Querschnitts und/oder Kanten der Arthrodeseplatte Fasen (18) und/oder Rundungen aufweisen.

7. Arthrodeseplatte (2) nach einem der vorhergehenden Ansprüche, **dadurch gekennzeichnet, dass** das Volumen der beiden Verankerungsbereiche (4, 6) in proximal-distaler Richtung sich im Wesentlichen geradlinig erstreckt oder dass die beiden Verankerungsbereiche in einem Winkel zueinander orientiert sind.

8. Arthrodeseplatte (2) nach Anspruch 7, **dadurch gekennzeichnet, dass** der Winkel 1° bis 50° beträgt, oder 5° bis 45°, oder 30°.

9. Arthrodeseplatte (2) nach Anspruch 7 oder 8, **dadurch gekennzeichnet, dass** zwischen den beiden Verankerungsbereichen (4, 6) eine schwenkbare und arretierbare Verbindung ausgebildet ist, die den Winkel einstellbar macht.

10. Arthrodeseplatte (2) nach Anspruch 9, **dadurch gekennzeichnet, dass** die arretierbare Verbindung komplementäre, aufeinander liegende Kontaktflächen aufweist, insbesondere mit um eine Schwenkachse sternförmigen Texturen, und/oder formschlüssig und/oder reibschlüssig arretierbar ist.

## Claims

1. An arthrodesis plate (2) for joint fusion, wherein the arthrodesis plate comprises:
a proximal-distal direction, a longitudinal direction in the proximal-distal direction, and a medio-lateral direction,
a proximal anchoring region (4) and a distal anchoring region (6) which are mechanically and rigidly connected to each other, wherein a transversal cross-section of the anchoring regions has a height (h) relative to the longitudinal direction and has a width (b) in the medio-lateral direction, wherein the width is greater than the height,
**characterized in that**
the proximal anchoring region (4) and the distal anchoring region (6) have an outer contour (10, 12) with an undulating cross-section in a plane parallel to the sagittal plane and/or in a transversal plane, and
**in that** the anchoring regions (4, 6) can be introduced in a medio-lateral direction into a planar slot (17), wherein the undulating cross-section is arranged to press with its crests into the boundary surfaces of the planar slot.

2. The arthrodesis plate (2) according to claim 1, **characterized in that** the upper side and the lower side of the anchoring regions (4, 6) have an undulating sagittal and/or transversal cross-section.

3. The arthrodesis plate (2) according to claim 2, **characterized in that** the undulating sagittal and/or transversal cross-section of the upper side and of the lower side run parallel to one another at least in portions.

4. The arthrodesis plate (2) according to any one of the preceding claims, **characterized in that** the undulating sagittal and/or transversal cross-section is zigzag-shaped with straight ascending flanks and straight descending flanks.

5. The arthrodesis plate (2) according to any one of the preceding claims, **characterized in that** the transversal cross-section is rectangular.

6. The arthrodesis plate (2) according to claim 5, **characterized in that** corners of the rectangular transversal cross-section and/or edges of the arthrodesis plate have chamfers (18) and/or roundings.

7. The arthrodesis plate (2) according to any one of the preceding claims, **characterized in that** the volume of both anchoring regions (4, 6) in the proximal-distal direction extends in a substantially straight line or **in that** the two anchoring regions are oriented at an angle to one another.

8. The arthrodesis plate (2) according to claim 7, **characterized in that** the angle is 1° to 50°, or 5° to 45°, or 30°.

9. The arthrodesis plate (2) according to claim 7 or 8, **characterized in that**, between both anchoring regions (4, 6), a pivotable and lockable connection is formed, which makes the angle adjustable.

10. The arthrodesis plate (2) according to claim 9, **characterized in that** the lockable connection has complementary contact surfaces lying one on top of the other, in particular with star-shaped textures about a pivot axis, and/or which can be locked in a form-fitting and/or frictionfitting manner.

## Revendications

1. Plaque d'arthrodèse (2) pour l'ankylose articulaire, dans laquelle la plaque d'arthrodèse comporte :
une direction proximale-distale, une direction longitudinale dans la direction proximale-distale et une direction médio-latérale,
une zone d'ancrage proximale (4) et une zone d'ancrage distale (6) mécaniquement reliées rigidement l'une à l'autre, dans laquelle une section transversale des portions d'ancrage présente une hauteur (h) dans la direction longitudinale et une largeur (b) dans la direction médio-latérale, dans laquelle la largeur est supérieure à la hauteur,
**caractérisée en ce**
**que** la zone d'ancrage proximale (4) et la zone d'ancrage distale (6) présentent un contour extérieur (10, 12) avec une section transversale ondulée dans un plan parallèle au plan sagittal et/ou dans un plan transversal, et
**que** les zones d'ancrage (4, 6) peuvent être introduites dans une fente plane (17) dans la direction médio-latérale, dans laquelle la section transversale ondulée est conçue pour s'appuyer avec ses crêtes d'onde dans les surfaces limites de la fente plane.

2. Plaque d'arthrodèse (2) selon la revendication 1, **caractérisée en ce que** la face supérieure et la face inférieure des zones d'ancrage (4, 6) ont une section sagittale et/ou transversale ondulée.

3. Plaque d'arthrodèse (2) selon la revendication 2, **caractérisée en ce que** la section sagittale et/ou transversale ondulée de la face supérieure et de la face inférieure sont parallèles entre elles au moins par tronçons.

4. Plaque d'arthrodèse (2) selon l'une quelconque des revendications précédentes, **caractérisée en ce que** la section sagittale et/ou transversale ondulée est en forme de zigzag avec des flancs droits ascendants et des flancs droits descendants.

5. Plaque d'arthrodèse (2) selon l'une quelconque des revendications précédentes, **caractérisée en ce que** la section transversale est rectangulaire.

6. Plaque d'arthrodèse (2) selon la revendication 5, **caractérisée en ce que** les coins de la section transversale rectangulaire et/ou les bords de la plaque d'arthrodèse présentent des chanfreins (18) et/ou des courbes.

7. Plaque d'arthrodèse (2) selon l'une quelconque des revendications précédentes, **caractérisée en ce que** le volume des deux zones d'ancrage (4, 6) dans la direction proximale-distale s'étend sensiblement en ligne droite ou que les deux zones d'ancrage sont orientées en un angle l'une par rapport à l'autre.

8. Plaque d'arthrodèse (2) selon la revendication 7, **caractérisée en ce que** l'angle est de 1° à 50°, ou de 5° à 45°, ou de 30°.

9. Plaque d'arthrodèse (2) selon la revendication 7 ou 8, **caractérisée en ce qu'**une liaison pivotante et verrouillable est formée entre les deux zones d'ancrage (4, 6), laquelle liaison rend l'angle réglable.

10. Plaque d'arthrodèse (2) selon la revendication 9, **caractérisée en ce que** la liaison verrouillable présente des surfaces de contact complémentaires superposées, en particulier avec des textures en étoile autour d'un axe de pivotement, et/ou verrouillables par complémentarité de forme et/ou par friction.
